Europäisches Patentamt

European Patent Office

Office européen des brevets

(19)

(11) Veröffentlichungsnummer : **0 031 437**
**B1**

(12) **EUROPÄISCHE PATENTSCHRIFT**

(45) Veröffentlichungstag der Patentschrift :
30.03.83

(51) Int. Cl.³ : **C 07 C 69/82**, C 07 C 67/00,
B 01 J 23/90

(21) Anmeldenummer : **80107094.7**

(22) Anmeldetag : **15.11.80**

(54) **Gewinnung und Wiederverwendung von Schwermetalloxidationskatalysator aus dem Witten-DMT-Prozess.**

(30) Priorität : **14.12.79 DE 2950318**
**01.10.80 DE 3037054**

(43) Veröffentlichungstag der Anmeldung :
**08.07.81 Patentblatt 81/27**

(45) Bekanntmachung des Hinweises auf die Patenterteilung : **30.03.83 Patentblatt 83/13**

(84) Benannte Vertragsstaaten :
**AT BE DE FR GB IT NL**

(56) Entgegenhaltungen :
DE A 2 811 757
DE B 2 531 106
DE B 2 923 681
FR A 2 162 570

(73) Patentinhaber : **DYNAMIT NOBEL AKTIENGESELL-
SCHAFT
Patentabteilung Postfach 1209
D-5210 Troisdorf, Bez. Köln (DE)**

(72) Erfinder : **Diessel, Karl-Heinz, Dr.
Düsseldorfer Strasse 28
D-3070 Nienburg (DE)**
Erfinder : **Modic, Rudolf, Dr.
Waldfrieden 8
D-3074 Steyerberg (DE)**
Erfinder : **Struss, Friedrich
Mozartstrasse 18
D-3073 Liebenau (DE)**

## Gewinnung und Wiederverwendung von Schwermetalloxidationskatalysator aus dem Witten-DMT-Prozeß

Die Erfindung betrifft ein Verfahren zur Gewinnung und Wiederverwendung von Schwermetalloxidationskatalysator aus dem Witten-DMT-Prozeß ausgehend von hochsiedenden Destillationsrückständen, mit einem Kobaltgehalt von 1 bis 10 g/kg und ggf. einem Mangangehalt von 0,1 bis 5 g/kg Rückstand und/oder einem Nickelgehalt von 0,1 bis 5 g/kg Rückstand, die bei der Oxidation von p-Xylol (PX) und/oder p-Toluylsäuremethylester (PTE) enthaltenden Gemischen in flüssiger Phase mit sauerstoffhaltigen Gasen bei einem Druck von 4 bis 8 bar und einer Temperatur von 140 bis 170 °C in Gegenwart von gelöstem Schwermetalloxidationskatalysator, anschließenden Veresterung des Oxidationsproduktes mit Methanol bei einem Druck von 20 bis 30 bar und einer Temperatur von 230 bis 280 °C und destillativen Auftrennung des Veresterungsproduktes in eine p-Toluylsäuremethylester-(PTE)-reiche Fraktion, eine Dimethylterephthalat-(DMT-)reiche Fraktion und einen hochsiedenden Destillationsrückstand anfallen, durch Extraktion des Schwermetalloxidationskatalysators mit Wasser, verdünnten Mineralsäuren, wässrigen niedermolekularen aliphatischen Monocarbonsäuren oder Alkoholen in der Wärme, ggf. nach Verbrennung der hochsiedenden Destillationsrückstände. DMT wird als Rohstoff zur Herstellung von Polyester durch Umsetzung mit Äthylenglykol oder Tetramethylenglykol für Fasern, Filaments, Filme oder Formteile benötigt. Es wird in zahlreichen großtechnischen Anlagen nach dem als Witten- oder auch Witten-Hercules-Verfahren bekannt gewordenen Prozeß hergestellt.

Technisch wird so gearbeitet, daß das PX- und PTE-haltige Gemisch in Abwesenheit von Lösungsmitteln und Halogenverbindungen in Gegenwart von im Reaktionsgemisch gelösten Kobalt- und Manganverbindungen zu einem überwiegend aus p-Toluylsäure (PTS), Monomethylterephthalat (MMT) und Terephthalsäure (TPS) bestehenden Oxidat umgesetzt und das Oxidat bei 230 bis 280 °C und 20 bis 30 bar mit Methanol verestert wird. Das Schwermetalloxidationskatalysatorsystem wird bevorzugt mit Mengen bezogen auf die Oxidatmenge und auf Metallgehalt umgerechnet von etwa 70-200 ppm Kobalt und 7-100 ppm Mangan eingesetzt. Das Veresterungsprodukt wird in einer sogenannten Rohesterdestillation destillativ in eine PTE-reiche Fraktion, eine DMT-reiche Fraktion sowie in einen hochsiedenden Destillationsrückstand aufgetrennt. Die PTE-reiche Fraktion wird der Oxidation, die DMT-reiche Fraktion einer nachfolgenden Reinigung und Aufarbeitung zugeführt. Der hochsiedende Rückstand enthält, neben den organischen Anteilen, die Verbindungen des Schwermetalloxidationskatalysatorsystems, z. B. Kobalt und Mangan.

Es ist technisch möglich, hochsiedende Destillationsrückstände der Oxidation von Alkylaromaten, aus denen keine weiteren Nutzprodukte mehr, sei es durch Isolierung oder durch Umwandlung gewonnen werden können, der Verbrennung, ggf. unter Nutzung der Verbrennungswärme zuzuführen, und die in den Rauchgasen der Verbrennung befindlichen schwermetallhaltigen Aschen über Zyklone oder Elektrofilter abzuscheiden (vgl. US-PS 3 341 470).

Der Schwermetallgehalt von Aschen der genannten Art setzt sich zusammen aus den in den schwersiedenden Destillationsrückständen noch vorhandenen Katalysatorbestandteilen, soweit diese nicht vorher, z. B. durch eine Extraktion entfernt worden sind, ferner aus den Werkstoffen der Herstellanlage durch Korrosion sowie den Bestandteilen der zugesetzten Brennstoffe der Rückstandsverbrennung, z. B. Masut oder schweres Heizöl.

Aschen der genannten Art können mit Mineralsäuren behandelt und die Schwermetalle als Karbonate oder Hydroxide aus den Lösungen ausgefällt werden (vgl. DE-OS 22 60 498). Die Abtrennung solcher Niederschläge durch Filtration oder Zentrifugieren sowie die Entfernung der anhaftenden entsprechenden Mineralsäure durch Auswaschen des Filterkuchens bereitet aber technische Schwierigkeiten.

Die völlige Entfernung der anorganischen Mineralsäurereste ist eine der Voraussetzungen für die Wiederverwendung der aus den schwersiedenden Rückständen stammenden Schwermetalle als Oxidationskatalysatoren bei der Oxidation von Alkylaromaten in flüssiger Phase mit Luftsauerstoff.

Ferner geht die Erfindung davon aus, daß in solchen Aschen der Verbrennung hochsiedender Destillationsrückstände des Herstellverfahrens für Alkylaromaten durch Oxidation in flüssiger Phase in Gegenwart von Schwermetalloxidationskatalysatoren sich Schwermetallbestandteile wie beispielsweise Eisen, Chrom, Vanadin, Molybdän, Kupfer und Titan anreichern, die aus den Werkstoffen der Herstellanlage und den Brennstoffen der Rückstandsverbrennung stammen und die die Aktivität des Kobalt-, Mangan- oder Nickelkatalysators bzw. von deren Gemischen bei der Rückführung in die Oxidationsreaktion erheblich reduzieren bzw. inhibieren.

Es ist für den DMT-Prozeß außerordentlich vorteilhaft, den Oxidationskatalysator, das ist ein Gemisch von Kobalt- und Manganverbindungen und/oder Nickelverbindungen aus diesem hochsiedenden Rückstand, ggf. nach Verbrennung des Rückstandes zurückzugewinnen und erneut zur Oxidation von PX und PTE einzusetzen.

Aufgabe der vorliegenden Erfindung ist es, aus den Destillationsrückständen der Rohesterdestillation die Katalysatorbestandteile zurückzugewinnen und aus dem Extrakt nach einer möglichst einfachen Aufarbeitung ohne Eindampfen oder durch die Anwesenheit von TMS und TMME erforderliche zusätzliche Maßnahmen direkt die für den Einsatz in die Oxidation oder eine anderweitige Verwendung der wertvollen Katalysatorbestandteile geeigneten wässrigen Lösungen zur Verfügung zu stellen.

Die Erfindung bezweckt, aus den sauren Extrakten ein Katalysatorregenerat zu erhalten, das von störenden organischen Bestandteilen, insbesondere TMS und TMME, ferner von Metallverbindungen aus den Werkstoffen der Herstellanlage durch Korrosion weitgehend frei ist.

In Patentanmeldung P 29 23 681 wird ein Verfahren zur Rückgewinnung von Oxidationskatalysator aus dem katalysatorhaltigen Destillationsrückstand, der bei der DMT-Herstellung anfällt, und Wiederverwendung des rückgewonnenen Katalysators in der Oxidation vorgeschlagen mit dem Ziel, die Selektivität der Oxidation auf dem gleichen hohen Niveau wie beim Einsatz von Frischkatalysator zu halten. Dort wurde gezeigt, daß bei der Extraktion des katalysatorhaltigen Destillationsrückstandes der Rohesterdestillation Trimellithsäure (TMS) und Trimellithsäuremonomethylester (TMME) mit dem Katalysator herausgelöst werden und daß TMS und TMME bei Rückführung mit dem Katalysator in die Oxidation den Ablauf der Oxidationsreaktion erheblich beeinträchtigen können. Daher wird in dem vorgenannten Verfahren im Extrakt das Mengenverhältnis von TMS + TMME zu Schwermetalloxidationskatalysator auf einen Wert von höchstens 1,8 : 1 bemessen.

Erfindungsgemäß kann der Gehalt an TMS und TMME im Extrakt des Destillationsrückstandes um ein Mehrfaches, z. B. fünfmal höher sein, als der Gehalt an Kobalt-Mangan und somit fast das Dreifache des in Patentanmeldung P 29 23 681 zugelassenen Verhältnisses von TMS + TMME zu Schwermetalloxidationskatalysator betragen.

Der Gehalt an TMS und TMME im Extrakt ist abhängig von der Art der Rohesteraufarbeitung und damit der chemischen Zusammensetzung des hochsiedenden Destillationsrückstandes. Bei steigendem Gehalt an TMS und TMME im Extrakt ist ein erhöhter Verbrauch an Schwermetalloxidationskatalysator erforderlich, um einen einwandfreien Ablauf der Oxidationsreaktion bei Rückführung des extrahierten Katalysators zu gewährleisten.

Ferner gestattet die Erfindung die Gewinnung und Wiederverwendung von Kobalt- oder Kobalt- und Manganverbindungen gemeinsam mit Nickelverbindungen.

Diese Aufgaben werden mit der Erfindung gelöst. Die Lösung besteht darin, daß bei einem Verfahren der angegebenen Art

a) der wässrige, saure schwermetalloxidationskatalysatorhaltige Extrakt mit einem Kobaltgehalt von 0,2 bis 20 g/l, ggf. einem Mangangehalt von 0,05 bis 10 g/l und ggf. einem Nickelgehalt von 0,05 bis 10 g/l mit einem stark sauren Kationenaustauscherharz in der Alkalimetallform, z. B. $Na^+$- oder $K^+$-Form bei erhöhter Temperatur bis zum Erreichen der Austauscherkapazität behandelt,

b) das Kationenaustauscherharz anschließend bei erhöhter Temperatur gewaschen und bei Raumtemperatur mit $Na^+$- oder $K^+$-acetathaltigen Lösungen unter Verdrängung der Katalysatorbestandteile und Erhalt einer wässrigen, essigsauren die Katalysatorbestandteile enthaltenden Lösung regeneriert und

c) die Regeneratkatalysatorlösung direkt in die Oxidation von p-Xylol und/oder p-Toluylsäuremethylester wiedereingesetzt wird.

Die schwermetallhaltigen Rückstände der Verbrennung der hochsiedenen Destillationsrückstände werden in Mineralsäuren, z. B. Salzsäure unter Zusatz von Oxidationsmitteln, z. B. Wasserstoffperoxid-Lösung zur Oxidation von u. a. $Fe^{2+}$-lonen gelöst und die in der Lösung angereicherten Verunreinigungen, die aus den Werkstoffen der Anlage und den Brennstoffen stammen, wie beispielsweise Eisen, Chrom, Vanadin, Molybdän, Kupfer und Titan durch Einstellen der Lösung auf einen pH-Wert von etwa 6 oder darüber mit beispielsweise wässriger Natronlauge als Hydroxide ausgefällt und zusammen mit den unlöslichen Anteilen der Asche abfiltriert. Die so gereinigte Lösung wird durch Zugabe von linearen niedermolekularen aliphatischen Monocarbonsäuren mit 1-4 C-Atomen, beispielsweise Essigsäure, auf einen pH-Wert von 5 oder darunter eingestellt. Die in der Lösung enthaltenen $Na^+$-lonen werden durch Behandlung mit einem $Co^{2+}$-, $Mn^{2+}$- und/oder $Ni^{2+}$-lonen beladenen stark sauren Kationenaustauscherharz entfernt. Die essigsaure Schwermetallösung wird anschließend mit einem stark sauren Kationenaustauscherharz in der $Na^+$- oder $K^+$-Form bis zum Erreichen der Austauscherkapazität behandelt und das Kationenaustauscherharz anschließend bei erhöhter Temperatur gewaschen und bei Raumtemperatur mit $Na^+$- oder $K^+$-acetathaltigen Lösungen unter Verdrängung der Metallionen der Katalysatorbestandteile und Erhalt einer wässrigen, essigsauren, die Metallionen der Katalysatorbestandteile enthaltenden Lösung regeneriert. Als Kationenaustauscher dient ein Kationenaustauscherharz, z. B. ein mit Divinylbenzol vernetztes Polystyrolgerüst, das Sulfonsäuregruppen als stark saure aktive Gruppen trägt.

Mit der erfindungsgemäßen Aufarbeitung der Verbrennungsprodukte der schwersiedenden Destillationsrückstände ist es auf einfache Art möglich, eine von Mineralsäureresten und die Aktivität der Katalysatorbestandteile inhibierenden Verunreinigungen freie wasserlösliche organische Katalysatorlösung zu erhalten.

Die erhaltenen wässrigen Katalysatorlösungen enthalten Kobalt- und Manganacetat mit einem Gehalt von etwa 5 bis 70 g/l Kobalt, 1 bis 35 g/l Mangan und ggf. Nickelacetat mit einem Gehalt von etwa 1 bis 35 g/l Nickel.

Diese wässrigen, die Katalysatorbestandteile als Acetate enthaltenden Lösungen werden mit Vorteil direkt wieder in die Oxidation der p-Xylol- und/oder p-Toluylsäuremethylesterhaltigen Gemische zurückgeführt.

3

Neben den Kationen der Alkalimetallgruppe, vorzugsweise Natrium oder Kalium, kann der Ionenaustauscher zur Absorption der Katalysatormetallbestandteile auch in der $H^+$-Ionenform eingesetzt werden.

Bevorzugt ist, den mit Katalysatormetallionen beladenen Austauscher mit verdünnten wässrigen Natriumacetatlösungen zu regenerieren, weil bei den erfindungsgemäß eingesetzten stark sauren Ionenaustauschern das Regenerationsgleichgewicht $R\text{-}(Co^{2+}) + 2\,Na^+ \rightleftarrows R\text{-}(Na^+)_2 + Co^{2+}$ im Vergleich zum Regenerationsgleichgewicht $R\text{-}(Co^{2+}) + 2\,H^+ \rightleftarrows 5\,R\text{-}(H^+)_2 + Co^{2+}$, wobei R die stationäre Ionenaustauschermatrix bedeutet, mehr in Richtung der rechten Seite der Reaktionsgleichung liegt.

Mit der erfindungsgemäßen Behandlung der erhaltenen Extrakte mit stark sauren Kationenaustauschern ist eine Aufkonzentration des Katalysatormetallgehaltes im Extrakt auf Werte bis etwa zum 20-fachen der Eingangskonzentration auf überraschend einfache Weise möglich, ohne daß vergleichsweise hohe Gehalte an TMS und TMME durch Komplexbildung den Austausch der Katalysatormetallionen durch das Gegenion, z. B. Natriumion am Austauscher stören.

Ein Einengen des Extraktes, durch Eindampfen, das bei erhöhten TMS- und TMME-Konzentrationen zu Verlusten an Katalysatormetall durch Ausfällungen und Niederschläge führen würde, kann erfindungsgemäß entfallen.

Vielmehr wird auf einfache Weise eine quantitative Abtrennung von TMS und TMME sowie anderer begleitender organischer Stoffe erreicht. Angesichts der Störungen, die erhebliche Gehalte an TMS und TMME in der Oxidation von PX- und/oder PTE-haltigen Mischungen verursachen, ist dieses Ergebnis von besonderem Wert.

Das erfindungsgemäße Verfahren wird technisch in einer bevorzugten Ausführung so durchgeführt, daß die sauren, wässrigen Extrakte von einer Temperatur von etwa 95 °C auf etwa Raumtemperatur abgekühlt und die dabei ausfallenden organischen Bestandteile abgetrennt werden. Es folgt Wiedererwärmung auf etwa 70 °C, um Nachfällungen zu vermeiden. Bei der anschließenden Beladung des stark sauren Kationenaustauschers in der $Na^+$-Form bei etwa 70 °C werden im Extrakt noch vorhandene Mengen organischer Bestandteile insbesondere TMS, TMME, TPS, MMT und dergleichen, nicht am Austauscher adsorbiert, sondern bleiben in der wässrigen Phase und passieren den Austauscher ungehindert. Für den Fall, daß dieses hauptsächlich mit Alkalimetallionen und organischen Verbindungen belastete Abwasser nicht einer biologischen Aufarbeitung zugeführt werden kann, sondern thermisch behandelt werden muß, können die Alkalimetallionen durch Behandlung mit einem stark sauren Kationenaustauscher in der $H^+$-Ionenform ausgetauscht und durch Eluieren mit einer starken Säure, vorzugsweise Salzsäure, als Neutralsalz, z. B. NaCl, in wässriger Lösung entfernt werden. Bei Erreichen der Austauschkapazität mit $Co^{2+}$ und $Mn^{2+}$ wird der beladene Austauscher mit auf ebenfalls etwa 70 °C erwärmtem vollentsalztem Wasser als Waschflüssigkeit behandelt. Diese Behandlung dient der Entfernung der am Austauscherharz absorbierten organischen Bestandteile, die sich als schmieriger Film auf der Austauscherharzmatrix niederschlagen und die Austauscherkapazität erheblich reduzieren würden, wenn sie nicht bei jedem Zyklus entfernt würden. Das anfallende Waschwasser wird zweckmäßig in die Extraktion zurückgeführt.

Bei der nachfolgenden Regeneration, die bei Raumtemperatur durchgeführt wird, werden etwa zwei Bettvolumina einer wässrigen Natriumacetatlösung, die bei dem vorhergehenden Regenerationszyklus als Nachlauf aufgefangen und mit dem Vorlauf des vorhergehenden Regenerationszyklus vereinigt wurde, auf den mit $Co^{2+}$ und $Mn^{2+}$ beladenen Austauscher, dessen Bettvolumen zunächst mit vollentsalztem Waschwasser ausgefüllt ist, aufgegeben. Etwa 50 % des Bettvolumens werden als $Co^{2+}/Mn^{2+}$-freie Lösung abgezogen. Anschließend werden etwa 15 % des Bettvolumens als schwach $Co^{2+}$-/$Mn^{2+}$-haltiger Vorlauf aufgefangen. Als nächste Fraktion werden etwa 135 % des Bettvolumens als $Co^{2+}$-/$Mn^{2+}$-Acetatlösung (im nachfolgenden als Konzentrat bezeichnet) abgenommen. Zur restlosen Verdrängung der Katalysatormetallbestandteile vom Austauscher werden etwa 80 % des Bettvolumens einer etwa 15 %igen wässrigen Natriumacetatlösung, die ca. 10 bis 15 g/l freie Essigsäure enthält, und darauf folgend etwa 80 % des Bettvolumens voll entsalztes Wasser zum Entfernen der $Co^+$ und $Mn^+$-Ionen enthaltenden Natriumacetatlösung auf den Austauscher gegeben. Die erhaltenen Lösungen in einer Gesamtmenge von etwa 160 % des Bettvolumens werden bis zum deutlichen Abfall der $Co^{2+}$-/$Mn^{2+}$-Konzentration in einer Menge von etwa 5 bis 10 % des Bettvolumens abgezogen und mit dem Konzentrat vereinigt und die folgenden Fraktionen in einer Menge von etwa 150 bis 155 % des Bettvolumens als Nachlauf abgezogen, mit dem vorher angefallenen Vorlauf vereinigt und zur Verwendung im folgenden Regenerationszyklus zurückgestellt.

Die folgenden Beispiele dienen zur weiteren Erläuterung der Erfindung.

Der in den Beispielen 3 und 4 eingesetzte Verbrennungsrückstand wurde durch Verbrennen eines hochsiedenden Destillationsrückstandes aus dem Witten-DMT-Verfahren bei 800-1 200 °C und Abscheidung in einem Elektrofilter aus den Rauchgasen erhalten.

Dabei wurden 95 Gew.-% eines solchen Rückstandes unter Zusatz von 5 Gew.-% schweren Heizöls verbrannt.

Der der Verbrennung zugeführte schwersiedende Destillationsrückstand enthielt etwa 0,1 bis 1,0 Gew.-% an aschebildenden Schwermetallbestandteilen.

## Beispiel 1

100 kg Destillationsrückstand aus der Rohesterdestillation, der in einer industriellen Anlage zur DMT-Herstellung durch gemeinsame kontinuierliche Oxidation von PX- und PTE-haltigen Gemischen in flüssiger Phase mit Luftsauerstoff bei 8 bar Druck und Temperaturen von 150 bis 170 °C unter Verwendung einer Lösung von Kobaltacetat und Manganacetat in wässriger Essigsäure, wobei im Oxidationsprodukt eine stationäre Konzentration von etwa 90 ppm Kobalt und 10 ppm Mangan eingestellt wird, anschließende kontinuierliche Veresterung des Oxidationsproduktes bei Temperaturen von etwa 250 °C und 25 bar Druck mit Methanol und kontinuierliche Auftrennung des Veresterungsproduktes durch Vakuumdestillation, wobei in einer ersten Destillationskolonne eine PTE-reiche Fraktion über Kopf abgenommen wird, die in die Oxidation zurückgeführt wird, und das Sumpfprodukt dieser Kolonne in einer zweiten nachgeschalteten Kolonne in eine DMT-reiche Fraktion, die über Kopf abgenommen wird, und einen schwersiedenden Destillationsrückstand mit einem Kobaltgehalt von 2,3 g/kg und einem Mangangehalt von 0,2 g/kg Rückstand aufgetrennt wurde, erhalten wurde, wurden mit 60 l Reaktionswasser der DMT-Produktion mit einem Säuregehalt von etwa 3 %, als Essigsäure berechnet, bei ca. 95 °C bis auf einen Rest-$Co^{2+}$-Gehalt von 20 ppm extrahiert.

Es wurden nach dem Dekantieren 56 l eines $Co^{2+}/Mn^{2+}$-haltigen Extraktes mit einem $Co^{2+}$-Gehalt von 3,8 g/l und einem $Mn^{2+}$-Gehalt von 0,3 g/l erhalten. Der heiße Extrakt wurde auf 20 °C abgekühlt und ausgefallene organische Stoffe durch Filtration abgetrennt.

Die filtrierte Lösung wurde auf 70 °C zur Verhinderung von Nachfällungen aufgeheizt und von unten durch ein mit einem $Na^+$-Ionen-beladenen stark sauren Kationenaustauscher mit dem Handelsnamen « Lewatit S 100 » gefülltes Rohr geleitet. Das Harzvolumen betrug 1,1 l. Die Beladung wurde bis zur beginnenden Erschöpfung der Ionenaustauscherkapazität fortgeführt.

Von den hergestellten 56 l Co-Mn-Extrakt wurden 19 l, entsprechend einem Gesamtgehalt von 78,4 g $Co^{2+}$ + $Mn^{2+}$ oder 2,66 Val $Co^{2+}$, bei 70 °C über den Kationenaustauscher geleitet. Anschließend wurde der Kationenaustauscher geleitet. Anschließend wurde der Kationenaustauscher mit 1 l voll entsalztem Wasser von oben bei 70 °C gewaschen. Darauf schloß sich das Eluieren des Kationenaustauschers von oben mit 2,2 l einer 10 %igen wässrigen essigsauren Natriumacetatlösung, entsprechend 1,3 Val $Na^+$/l, und anschließendem Nachwaschen mit 1 l voll entsalztem Wasser bei Raumtemperatur an. Es wurden 0,4 l Vorlauf, 2,0 l Konzentrat und 0,8 l Nachlauf erhalten.

Das wässrige Konzentrat enthielt folgende Bestandteile :

| | |
|---|---|
| $Co^{2+}$ = | 30,1 g/l |
| $Mn^{2+}$ = | 2,6 g/l |
| $Na^+$ = | 1,0 g/l |
| $CH_3COOH$ = | 12,0 g/l |
| organische Verunreinigungen | nicht feststellbar (polarografisch) |

Der erhaltene $Co^{++}$-, $Mn^{++}$- und der $Na^+$-haltige Vor- bzw. Nachlauf wurden vereinigt und erneut als Elutionslösung eingesetzt, um $Co^{++}$ und $Mn^{++}$-Verluste zu vermeiden.

## Beispiel 2

In einer kontinuierlich arbeitenden Extraktionsanlage wurden 300 kg/h des hochsiedenden Destillationsrückstandes der Rohesterdestillation, der wie in Beispiel 1 erhalten wurde, bei ca. 95 °C mit 150 kg/h saurem Reaktionswasser aus der DMT-Produktion, dessen Herkunft und Säuregehalt entsprechend dem Beispiel 1 war, unter Rühren extrahiert.

Die nach Abtrennung von der organischen Phase erhaltene wässrige Lösung enthielt 4,6 g/l $Co^{2+}$ und 0,4 g/l $Mn^{2+}$. Diese wurde auf etwa 20 °C abgekühlt und durch Filtration von den ausgefallenen organischen Produkten, die in den Prozeß zurückgeführt wurden, getrennt und in einem Behälter gesammelt. Nach Erhitzen auf etwa 70 °C, um ein nachträgliches Ausfallen organischer Stoffe zu vermeiden, wurden 650 l/std. dieser Lösung bei einer Temperatur von 70 °C über eine Säule, die mit 180 l eines mit $Na^+$-Ionen beladenen Harzes mit dem Handelsnamen « Lewatit S 100 » beschickt war, geleitet. Die Beladung des Austauschers war nach etwa 3 Stunden beendet.

Danach wurde zur Entfernung organischer Verbindungen mit 400 l heißem, vollentsalztem Wasser gespült. Anschließend wurden die Katalysatorionen mit einer natriumacetathaltigen Lösung mit einem Gehalt von 10-15 g/l freier Essigsäure, die zum Teil aus den Vor- und Nachläufen der vorhergehenden Elution sowie einer 15 %igen Natriumacetatlösung bestand, bei etwa Raumtemperatur eluiert. Insgesamt wurden zur Elution 515 l Lösung eingesetzt.

Nach dem Eluieren wurde der Austauscher mit 170 l vollentsalztem Wasser gewaschen.

Es wurden vier Fraktionen aufgefangen : 90 l $Co^{++}$ und $Mn^{++}$ freie Lösung, 35 l Vorlauf, 240 l Konzentrat und 320 l Nachlauf.

Das Konzentrat enthielt

37,0 g/l $Co^{++}$ und

5

3,1 g/l Mn$^{++}$.

Organische Bestandteile außer Essigsäure waren polarografisch nicht nachweisbar. Vor- und Nachlauf wurden vereinigt und erneut für den nächsten Zyklus verwendet. Das Konzentrat wurde direkt in die im Beispiel 1 beschriebene Oxidation wiedereingesetzt. Die Aktivität dieses Konzentrats war identisch mit der von Frischkatalysatorlösung gleicher Kobalt- und Manganacetatkonzentration.

### Beispiel 3

50,5 g eines Verbrennungsrückstandes aus dem DMT-Prozeß wurden unter Rühren mit 300 ml verdünnter HCl-Lösung (= 12 % HCl) und 2 ml 30 %iger $H_2O_2$-Lösung zwei Stunden bei 95 °C aufgeschlossen.

Die verwendete Probe enthielt :

|        |      |           |
|--------|------|-----------|
| 50,7   | %    | Kobalt    |
| 5,4    | %    | Mangan    |
| 0,37   | %    | Eisen     |
| 0,13   | %    | Nickel    |
| 100    | ppm  | Chrom     |
| 1 000  | ppm  | Molybdän  |
| 100    | ppm  | Vanadium  |
| 100    | ppm  | Kupfer    |
| 100    | ppm  | Titan.    |

Anschließend wurde der Aufschluß mit 1 l vollentsalztem (VE-)Wasser verdünnt und mit ca. 40 %iger Natronlauge bis pH 7 versetzt. Verbraucht wurden 9 ml Natronlauge. Der Aufschluß wurde dann eine Stunde auf 95 °C erwärmt und durch ein Faltenfilter filtriert.

Das Filtrat wurde nach Verdünnung mit VE-Wasser auf 8 Liter und mit 5 ml konzentrierter Essigsäure auf pH 4 eingestellt.

Die Lösung enthielt :

| 2,9 g | Kobalt/l |
|-------|----------|
| 0,2 g | Mangan/l |
| 6 ppm | Nickel   |
| <5 ppm | Eisen |
| <5 ppm | Chrom, Molybdän, Vanadium, Kupfer, Titan. |

Die erhaltene Lösung wurde zur weitgehenden Entfernung der Na$^+$-Ionen durch eine Säule mit 250 ml eines mit Co$^{2+}$- und Mn$^{2+}$-Ionen beladenen stark sauren Kationenaustauscherharzes geleitet.

Eine Säule mit 250 ml stark saurem Kationenaustauscherharz Lewatit S 100 in der Na$^+$-Form wurde mit der Lösung beladen.

Das am Ablauf erhaltene Abwasser enthielt :

30 ppm Kobalt
2 ppm Mangan.

Der Austauscher wurde bis zur beginnenden Erschöpfung mit 3,5 l obiger Lösung beladen (Grenzwert = 300 ppm Kobalt im Ablauf.)

Anschließend wurde der Austauscher mit 250 ml VE-Wasser gewaschen.

Die Elution der Kobalt- und Mangan-Ionen wurde mit folgenden Lösungen vorgenommen :

400 ml vereinigte Vor- und Nachlauf-Fraktionen vom vorhergehenden Versuch
200 ml einer 18 %igen Natriumacetat-Lösung mit 15 g freier Essigsäure/l
200 ml Ve-Wasser.

Erhalten wurden bei der Elution :

60 ml einer Vorlauf-Fraktion
400 ml einer Na$^+$-armen Hauptfraktion
340 ml einer Na$^+$-reichen Nachlauf-Fraktion.

Der Vor- und Nachlauf wurden vereinigt und zur Elution beim nächsten Versuch eingesetzt.

Die Hauptfraktion enthielt :

30,9 g Kobalt/l
2,1 g Mangan/l

75 ppm Nickel
< 5 ppm Chrom, Molybdän, Vanadium, Kupfer, Titan
175 ppm Natrium.

Die erhaltene Hauptfraktion kann als Katalysatorlösung in DMT-Prozeß verwendet werden.

## Beispiel 4

50,1 g eines Verbrennungsrückstandes wurden mit 350 ml 12 %iger Salzsäure und 2 ml 30 %iger $H_2O_2$-Lösung 2 Stunden bei 95 °C aufgeschlossen.
Der eingesetzte Ascherückstand enthielt :

40,6 % Co
4,3 % Mn
19,8 % Ni
2 970 ppm Fe
<100 ppm Cr
800 ppm Mo
< 100 ppm V
80 ppm Cu
< 100 ppm Ti
1 280 ppm Na.

Der Aufschluß wurde mit 7 ml einer ca. 40 %igen Natronlauge auf pH 6,2 eingestellt. Nach einer Stunde wurde die auf 95 °C erwärmte Lösung durch ein Faltenfilter filtriert.
Das Filtrat wurde auf 10 l mit VE-Wasser verdünnt und mit 10 ml konzentrierter Essigsäure auf pH 3,9 eingestellt.
Die Lösung enthielt :

1,84 g Co/l
0,16 g Mn/l
0,87 g Ni/l
< 5 ppm Fe
< 5 ppm Cr
< 5 ppm Mo
< 5 ppm V
< 5 ppm Cu
< 5 ppm Ti.

Die erhaltene Lösung wurde zur weitgehenden Entfernung der $Na^+$-Ionen mit 250 ml einem mit $Co^{2+}$-, $Mn^{2+}$- und $Ni^{2+}$-Ionen beladenen stark sauren Kationenaustauscherharzes behandelt.
Eine Säule mit 250 ml eines stark sauren Kationenaustauscherharzes LEWATIT S 100 in der $Na^+$-Form wurde mit der Lösung beladen.
Das am Ablauf erhaltene Abwasser enthielt :

25 ppm Co
2 ppm Mn
10 ppm Ni.

Zur Beladung des Austauschers bis zur beginnenden Erschöpfung wurden 4,7 l der Lösung verbraucht.
Der Austauscher wurde anschließend mit 250 ml VE-Wasser gewaschen.
Zur Elution der $Co^{2+}$-, $Mn^{2+}$- und $Ni^{2+}$-Ionen wurden folgende Lösungen ververwendet :

390 ml vereinigte Vor- und Nachlauf-Fraktion vom vorhergehenden Versuch
200 ml 18 %ige Natriumacetat-Lösung, die 15 g freie Essigsäure pro Liter enthielt
210 ml VE-Wasser.

Bei der Elution wurden folgende Lösungen erhalten :

80 ml einer Vorlauf-Fraktion
400 ml einer $Na^+$-armen Hauptfraktion
320 ml einer $Na^+$-reichen Nachlauf-Fraktion.

Die Hauptfraktion enthielt :

7

0 031 437

```
21,5 g    Co/l
 1,9 g    Mn/l
10,2 g    Ni/l
 < 5   ppm Fe
 < 5   ppm Cr
 < 5   ppm Mo
 < 5   ppm V
 < 5   ppm Cu
 < 5   ppm Ti
 240   ppm Na.
```

**Ansprüche**

1. Verfahren zur Gewinnung und Wiederverwendung von Schwermetalloxidationskatalysator aus dem Witten-Prozeß zur Herstellung von Dimethylterephthalat ausgehend von hochsiedenden Destillationsrückständen mit einem Kobaltgehalt von 1 bis 10 g/kg und ggf. einem Mangangehalt von 0,1 bis 5 g/kg Rückstand und/oder einem Nickelgehalt von 0,1 bis 5 g/kg Rückstand, die bei der Oxidation von p-Xylol und/oder p-Toluylsäuremethylester enthaltenden Gemischen in flüssiger Phase mit Sauerstoff oder einem Sauerstoff enthaltenden Gas bei einem Druck von 4 bis 8 bar und einer Temperatur von 140 bis 170 °C in Gegenwart von gelöstem Schwermetalloxidationskatalysator, anschließen den Veresterung des Oxidationsproduktes mit Methanol bei einem Druck von 20 bis 30 bar und einer Temperatur von 230 bis 280 °C und destillativen Auftrennung des Veresterungsproduktes in eine p-Toluylsäuremethylesterreiche Fraktion, eine Dimethylterephthalatreiche Fraktion und eine hochsiedenden Destillationsrückstand anfallen, durch Extraktion des Schwermetalloxidationskatalysators mit Wasser, verdünnten Mineralsäuren, wässrigen niedermolekularen aliphatischen Monocarbonsäuren mit 1-4 C-Atomen oder Alkoholen in der Wärme, ggf. nach Verbrennung der hochsiedenden Destillationsrückstände, dadurch gekennzeichnet, daß

a) der wässrige, saure, schwermetalloxidationskatalysatorhaltige Extrakt mit einem Kobaltgehalt von 0,2 bis 20 g/l ggf. einem Mangangehalt von 0,05 bis 10 g/l ggf. einem Nickelgehalt von 0,05 bis 10 g/l mit einem stark sauren Kationenaustauscherharz in der Alkalimetallform, z. B. Na$^+$- oder K$^+$-Form bei erhöhter Temperatur bis zur Erreichung der Austauscherkapazität behandelt,

b) das Kationenaustauscherharz anschließend bei erhöhter Temperatur gewaschen und bei Raumtemperatur mit Na$^+$- oder K$^+$-Acetathaltigen Lösungen unter Verdrängung der Katalysatorbestandteile und Erhalt einer wässrigen, essigsauren, die Katalysatorbestandteile enthaltenden Katalysatorlösung regeneriert und

c) die Regeneratkatalysatorlösung direkt in die Oxidation von p-Xylol und/oder p-Toluylsäuremethylester wiedereingesetzt wird.

2. Verfahren nach Anspruch 1, gekennzeichnet durch die Extraktion der nach Verbrennung der hochsiedenden Destillationsrückstände erhaltenen Aschen mit wässrigen Mineralsäuren unter Zusatz von Oxidationsmitteln in der Wärme, anschließendes Verdünnen mit Wasser und Erhöhung des pH-Wertes durch Zusatz von Alkali, Erwärmen zwecks Ausfällung von Eisen und Chrom als Hydroxide, gemeinsames Abfiltrieren der Hydroxide sowie unlöslichen Aschebestandteile, Verdünnen mit Wasser und Ansäuern des Filtrats mit linearen, niedermolekularen, aliphatischen Monocarbonsäuren mit 1-4 C-Atomen.

3. Verfahren nach Anspruch 2, dadurch gekennzeichnet, daß die Gewinnung der Katalysatorbestandteile aus den Aschen der Verbrennung der hochsiedenden Destillations rückstände durch Behandeln mit wässriger Salzsäure unter Zusatz von wässrigem H$_2$O$_2$ bei etwa 95 °C für den Zeitraum von 0,1-4 Stunden, anschließende Erhöhung des pH-Wertes auf einen Wert von 6 oder darüber durch Zusatz wässriger Alkalien, Erwärmen auf etwa 95 °C für 0,1 bis 2 Stunden, gemeinsames Abfiltrieren der gebildeten Hydroxide von Eisen, Chrom sowie der unlöslichen Aschebestandteile und Ansäuern des Filtrats mit Essigsäure auf pH 5 oder darunter und Entfernung der im Filtrat enthaltenen Na$^+$-Ionen mit Hilfes eines mit Co$^{2+}$, Mn$^{2+}$ und/oder Ni$^{2+}$-Ionen beladenen stark sauren Kationenaustauscherharzes erfolgt.

4. Verfahren nach Anspruch 2, dadurch gekennzeichnet, daß die Gewinnung von Kobalt- oder Kobalt- und Manganverbindungen gemeinsam mit Nickelverbindungen aus Extrakten von Aschen der Verbrennung der hochsiedenden kobalt-, kobalt- und manganhaltigen, kobalt- und nickelhaltigen oder kobalt-, mangan- und nickelhaltigen Destillationsrückstände mit einem Gehalt von 0,2 bis 20 g/l Kobalt, 0,05 bis 10 g/l Mangan bzw. 0,05 bis 10 g/l Nickel erfolgt.

5. Verfahren nach einem der Ansprüche 1 bis 4, dadurch gekennzeichnet, daß die gemäß Schritt b) von Anspruch 1 erhaltene Katalysatorlösung Kobalt- und Manganacetat mit einem Gehalt von 10 bis 70 g/l Kobalt und ggf. einem Gehalt von 1 bis 35 g/l Mangan, sowie weniger als jeweils 5 mg Eisen/l, 5 mg Chrom/l, 5 mg Molybdän/l, 5 mg Vanadium/l, 5 mg Kupfer/l und 5 mg Titan/l enthält.

6. Verfahren nach einem der Ansprüche 1 bis 4, dadurch gekennzeichnet, daß die gemäß Schritt b) von Anspruch 1 erhaltene Katalysatorlösung Kobalt-, Mangan- und Nickelacetat mit einem Gehalt von 10 bis 70 g/l Kobalt, 1 bis 35 g/l Mangan und 1 bis 35 g/l Nickel, sowie weniger als jeweils 5 mg Eisen/l, 5 mg Chrom/l, 5 mg Molybdän/l, 5 mg Vanadium/l, 5 mg Kupfer/l und 5 mg Titan/l enthält.

## Claims

1. Process for the recovery and reuse of heavy metal oxidation catalyst from the Witten process for the production of dimethyltherephthalate proceeding from high boiling distillation residues with a cobalt content of 1 to 10 g/kg and possibly a manganese content of 0.1 to 5 g/kg residue and/or a nickel content of 0.1 to 5 g/kg of residue, which accumulate in the oxidation of p-xylene and/or p-toluic acid methyl ester-containing mixtures in the liquid phase with oxygen or an oxygen-containing gas at a pressure of 4 to 8 bar and a temperature of 140 to 170 °C in the presence of dissolved heavy metal oxidation catalyst, subsequent esterification of the oxidation product with methanol at a pressure of 20 to 30 bar and a temperature of 230 to 280 °C and distillative separation of the esterification product into a p-toluic acid methyl ester-rich fraction, a dimethylterephthalate-rich fraction and a high boiling distillation residue, by extraction of the heavy metal oxidation catalyst with water, diluted mineral acids, aqueous low molecular aliphatic monocarboxylic acid with 1 to 4 C-atoms or alcohols with heating, optionally after combustion of the high boiling distillation residues, characterised in that

a) the aqueous, acidic, heavy metal oxidation catalyst-containing extract with a cobalt content of 0.2 to 20 g/l, where appropriate a manganese content of 0.05 to 10 g/l and where appropriate a nickel content of 0.05 to 10 g/l is treated with a strongly acid cation exchanger resin in alkali metal form, for example $Na^+$- or $K^+$-form at elevated temperature up until its exchanger capacity is attained,

b) the cation exchanger resin is washed at elevated temperature and is regenerated at room temperature with $Na^+$- or $K^+$-acetate-containing solutions with displacement of the catalyst components and receipt of an aqueous, acetic acid catalyst solution containing the catalyst components, and

c) the regenerated catalyst solution is employed again directly in the oxidation of p-xylene and/or p-toluic acid methyl ester.

2. Process according to claim 1, characterised by the extraction of the ashes obtained after combustion of the high-boiling distillation residues, using aqueous mineral acids with warming, in the presence of added oxidation means, subsequent dilution with water and increasing of the pH value by addition of alkali, warming for the purpose of precipitation of iron and chromium as hydroxides, combined filtering off of the hydroxides as well as insoluble ash components, dilution with water and acidification of the filtrate with linear low-molecular, aliphatic monocarboxylic acids with 1 to 4 C-atoms.

3. Process according to claim 2, characterised in that the recovery of the catalyst components from the ashes of the combustion of the high-boiling distillation residues takes place by treatment with aqueous hydrochloric acid with addition of aqueous $H_2O_2$ at about 95 °C for a period of 0.1 to 4 hours, subsequent increasing of the pH-value to a value of 6 or more by addition of aqueous alkalis, heating to about 95 °C for 0.1 to 2 hours, filtering off together of the hydroxides of iron and chromium which have formed, as well as of the insoluble ash components and acidification of the filtrate with acetic acid at a pH of 5 or below and removal of $Na^+$-ions existing in the filtrate with the aid of a strongly acidic cation exchanger resin charged with $Co^{2+}$, $Mn^{2+}$ and/or $Ni^{2+}$ ions.

4. Process according to claim 2, characterised in that the recovery of cobalt- or cobalt- and manganese-compounds together with nickel-compounds takes place from extracts of ashes from the combustion of the high boiling cobalt-, cobalt- and manganese-containing, cobalt- and nickel-containing or cobalt-, manganese- and nickel-containing distillation residues with a content of 0.2 to 20 g/l cobalt, 0.05 to 10 g/l manganese and 0.05 to 10 g/l nickel respectively.

5. Process according to one of claims 1 to 4, characterised in that the catalyst solution obtained according to step (b) of claim 1 contains cobalt and manganese acetate with a content of 10 to 70 g/l cobalt and where appropriate a content of 1 to 35 g/l manganese as well as, in each case, less than 5 mg iron/l, 5 mg chromium/l, 5 mg molybdenum/l, 5 mg vanadium/l, 5 mg copper/l and 5 mg titanium/l.

6. Process according to one of claims 1 to 4, characterised in that the catalyst solution obtained according to step b) of Claim 1 contains cobalt, manganese and nickel acetate with a content of 10 to 70 g/l cobalt, 1 to 35 g/l manganese and 1 to 35 g/l nickel as well as, less than in each case 5 mg/l iron, 5 mg/l chromium, 5 mg/l molybdenum, 5 mg vanadium/l, 5 mg copper/l and 5 mg titanium/l.

## Revendications

1. Procédé de préparation et de réutilisation d'un catalyseur d'oxydation de métaux lourds provenant du procédé de Witten pour la préparation de téréphtalate de diméthyle à partir de résidus de distillation à point d'ébullition élevé, ayant une teneur en cobalt de 1 à 10 g/kg et éventuellement une teneur en manganèse de 0,1 à 5 g/kg de résidus et/ou une teneur en nickel de 0,1 à 5 g/kg de résidus, résidus formés lors de l'oxydation de mélanges contenant du p-xylène et/ou l'ester méthylique d'acide p-toluylique en phase liquide, avec de l'oxygène ou des gaz contenant de l'oxygène sous une pression de 4 à 8 bars et à une température de 140 à 170 °C en présence d'un catalyseur d'oxydation de métaux lourds en solution, avec estérification ultérieure du produit d'oxydation avec du méthanol sous une pression de 20 à 30 bars et à une température de 230 à 280 °C et en séparant le produit d'estérification par distillation en une fraction riche en ester méthylique d'acide p-toluylique, une fraction riche en téréphtalate de diméthyle et en un résidu de distillation à point d'ébullition élevé, le catalyseur d'oxydation de métaux lourds étant soumis à une extraction avec de l'eau, des acides minéraux dilués, des acides monocarboxyliques aliphatiques aqueux de faible poids moléculaire contenant 1 à 4 atomes de carbone ou des alcools à chaud, éventuellement après combustion des résidus de distillation à point d'ébullition élevé, caractérisé en ce que

a) on traite l'extrait acide aqueux contenant le catalyseur d'oxydation de métaux lourds et ayant une teneur en cobalt de 0,2 à 20 g/l, éventuellement une teneur en manganèse de 0,05 à 10 g/l et éventuellement une teneur en nickel de 0,05 à 10 g/l, avec une résine échangeuse de cations fortement acide sous la forme d'un métal alcalin, par exemple, sous la forme $Na^+$ ou $K^+$, à température élevée et jusqu'à ce qu'on atteigne la capacité d'échange,

b) on lave ensuite la résine échangeuse de cations à température élevée et on la régénère à la température ambiante avec des solutions contenant de l'acétate de $Na^+$ ou de $K^+$ en balayant les constituants catalytiques et en obtenant une solution acétique aqueuse contenant les constituants catalytiques, et

c) on réutilise directement la solution du catalyseur régénéré lors de l'oxydation du p-xylène et/ou de l'ester méthylique d'acide p-toluylique.

2. Procédé suivant la revendication 1, caractérisé en ce qu'il comprend l'extraction des cendres obtenues après la combustion des résidus de distillation à point d'ébullition élevé avec des acides minéraux aqueux en ajoutant des agents d'oxydation à chaud, la dilution ultérieure avec de l'eau et l'élévation du pH par addition d'alcalis, le chauffage en vue de précipiter le fer et le chrome sous forme d'hydroxydes, la filtration commune des hydroxydes, ainsi que des constituants de cendres insolubles, la dilution avec de l'eau et l'acidification du filtrat avec des acides monocarboxyliques aliphatiques linéaires de faible poids moléculaire contenant 1 à 4 atomes de carbone.

3. Procédé suivant la revendication 2, caractérisé en ce qu'on obtient les constituants catalytiques à partir des cendres de la combustion des résidus de distillation à point d'ébullition élevé par traitement avec de l'acide chlorhydrique aqueux en ajoutant du $H_2O_2$ aqueux à une température d'environ 95 °C pendant une période de 0,1 à 4 heures, par élévation ultérieure du pH à une valeur de 6 ou plus en ajoutant des alcalis aqueux, par chauffage à environ 95 °C pendant 0,1 à 2 heures, par filtration commune des hydroxydes de fer et de chrome formés, ainsi que des constituants de cendres insolubles et par acidification du filtrat avec de l'acide acétique à un pH de 5 au moins, ainsi que par élimination des ions $Na^+$ contenus dans le filtrat à l'aide d'une résine échangeuse de cations fortement acide chargée d'ions $Co^{2+}$, $Mn^{2+}$ et/ou $Ni^{2+}$.

4. Procédé suivant la revendication 2, caractérisé en ce que l'obtention de composés de cobalt ou de cobalt et de manganèse a lieu conjointement avec des composés de nickel à partir d'extraits de cendres de la combustion de résidus de distillation à point d'ébullition élevé contenant du cobalt, du cobalt et du manganèse, du cobalt et du nickel ou du cobalt, du manganèse et du nickel avec une teneur de 0,2 à 20 g de cobalt/l, de 0,05 à 10 g de manganèse/l ou de 0,05 à 10 g de nickel/l.

5. Procédé suivant une des revendications 1 à 4, caractérisé en ce que la solution catalytique obtenue conformément à l'étape (b) de la revendication 1 contient de l'acétate de cobalt et de manganèse avec une teneur de 10 à 70 g de cobalt/l et éventuellement une teneur de 1 à 35 g de manganèse/l, ainsi que chaque fois moins de 5 mg de fer/l, 5 mg de chrome/l, 5 mg de molybdène/l, 5 mg de vanadium/l, 5 mg de cuivre/l et 5 mg de titane/l.

6. Procédé suivant une des revendications 1 à 4, caractérisé en ce que la solution catalytique obtenue conformément à l'étape (b) de la revendication 1 contient de l'acétate de cobalt, de manganèse et de nickel avec une teneur de 10 à 70 g de cobalt/l, 1 à 35 g de manganèse/l et 1 à 35 g de nickel/l, ainsi que chaque fois moins de 5 mg de fer/l, 5 mg de chrome/l, 5 mg de molybdène/l, 5 mg de vanadium/l, 5 mg de cuivre/l et 5 mg de titane/l.